# FASCICULE DE BREVET EUROPEEN

(11) **EP 1 257 599 B1**
(45) Date de publication et mention de la délivrance du brevet: **21.07.2004**
(21) Numéro de dépôt: 01907829.4
(22) Date de dépôt: 16.02.2001
(51) Int. Cl.: C08L 71/02, C08L 89/06, C08L 5/00, C08L 5/14, C11D 3/38, C11D 3/22, C11D 3/37, A61K 7/48, B01F 17/00

(54) **UTILISATION DE COMPOSITIONS A BASE DE PROTEINES, DE POLYSACCHARIDES ET DE DERIVES D'OXYDE D'ETHYLENE COMME SURFACTANTS**
VERWENDUNG VON ZUSAMMENSETZUNGEN ENTHALTEND PROTEINE, POLYSACCHARIDE UND ETHYLENOXIDDERIVATE ALS TENSIDMITTEL
USE OF COMPOSITIONS BASED ON PROTEINS, POLYSACCHARIDES AND ETHYLENE OXIDE DERIVATIVES AS SURFACTANTS

(30) Priorité: 17.02.2000 FR 0001952
(43) Date de publication de la demande: 20.11.2002
(73) Titulaire: Chalen Papier Europe Service, 60260 Lamorlaye (FR)
(72) Inventeur: CORBY, Charles, F-60260 Lamorlaye (FR); CORBY, Anne, F-60260 Lamorlaye (FR); CHERADAME, Hervé, F-92340 Bourg La Reine (FR)
(74) Mandataire: Corizzi, Valérie
(86) Numéro de dépôt international: PCT/FR2001/000470
(87) Numéro de publication internationale: WO 2001/060916

(56) Documents cités:
- EP-A- 0 194 097
- EP-A- 0 284 569
- US-A- 4 613 340
- DATABASE WPI Section Ch, Week 197905 Derwent Publications Ltd., London, GB; Class A85, AN 1979-08651B XP002151236 & JP 53 143971 A (HITACHI LTD), 14 décembre 1978 (1978-12-14)
- DATABASE WPI Section Ch, Week 198610 Derwent Publications Ltd., London, GB; Class D21, AN 1986-064925 XP002151235 & JP 61 015813 A (TOYO BEAUTY KK), 23 janvier 1986 (1986-01-23) cité dans la demande
- DATABASE WPI Section Ch, Week 198938 Derwent Publications Ltd., London, GB; Class A96, AN 1989-274241 XP002151237 & JP 01 199158 A (SEITETSU CHEM IND CO LTD), 10 août 1989 (1989-08-10)
- DATABASE EPODOC [en ligne] EUROPEAN PATENT OFFICE, THE HAGUE, NL; XP002172720 & CN 1 170 028 A (ZHOU JUN) 14 janvier 1998 (1998-01-14)

## Description

L'Invention se rapporte à l'utilisation de compositions à base de protéines. de polysaccharides et de dérivés d'oxyde d'éthylène, comme surfactants et, en particulier, comme détergents et dispersants.

Les détergents actuellement utilisés en milieu aqueux, tant dans le domaine ménager qu'industriel, sont constitués par 10 à 20% d'agents tensio-actifs du type alcane-sulfonates, alcools gras sulfatés à chaîne droite, éther-sulfates de sodium, ..., qui sont des composés synthétiques obtenus principalement à partir de dérivés de la pétrochimie (hydrocarbures) et de l'oléochimie, et qui leur confèrent l'essentiel de leurs propriétés détergentes, le reste étant représente par des adjuvants qui ont pour fonction de diluer ces agents tensio-actifs, de renforcer leur action et/ou de donner aux détergents des propriétés complémentaires.

Ces détergents ont, dans la plupart des cas, pour inconvénients majeurs :
- de présenter un pH nettement acide ou, au contraire, fortement alcalin. lequel est responsable d'un effet corrosif qui peut à la longue entraîner une détérioration, non seulement des objets nettoyés au moyen de ces détergents, mais également du matériel dans lequel s'effectue leur nettoyage (tambour de lave-linge par exemple) ou servant à l'évacuation des eaux usées.
- d'avoir souvent une forte tendance au moussage, qui est liée à la présence des agents tensio-actifs et qui, outre de se traduire parfois par des débordements indésirables de mousse, oblige généralement à procéder, en fin de nettoyage, à un ou plusieurs rinçages pour éliminer la mousse dans laquelle se trouvent emprisonnées les salissures,
- de présenter une aptitude au contact alimentaire faible, voir nulle, ce qui les rend dangereux en cas d'ingestion accidentelle ou volontaire, et justifie également la nécessité d'effectuer un ou plusieurs rinçages en fin de nettoyage, dès lors que les objets nettoyés sont destinés à être utilisés pour la préparation ou la consommation de produits alimentaires (vaisselle par exemple), et
- de représenter une charge notable pour l'environnement, même s'il est vrai que les fabricants de détergents ont fait des efforts considérables depuis le début des années 1960 pour développer des agents tensio-actifs hautement biodégradables et limiter l'emploi de certains adjuvants connus pour être polluants, comme les phosphates. Néanmoins, bon nombre de détergents disponibles à ce jour n'ont toujours pas une biodégradabilité optimale.

Les Inventeurs se sont, donc, fixés pour but de fournir une composition détergente qui, tout en assurant un nettoyage très efficace et ce, quelle que soit la nature des objets devant être nettoyés (textiles, vaisselle, ustensiles de cuisine, surfaces dures du type sols, murs ou plafonds, revêtements de sanitaires, appareillages industriels, ...) et leur état de saleté, soit dénuée des inconvénients précédemment mentionnés.

Les Inventeurs se sont, également, fixés pour but de fournir une composition détergente qui soit, de plus, dénuée de toute agressivité vis-à-vis des fibres textiles, de manière à pouvoir être utilisée pour le lavage du linge, y compris du linge délicat (soie, laine, ...).

Les Inventeurs se sont, encore, fixés pour but de fournir une composition détergente qui, outre de présenter tous ces avantages, puisse être commercialisée sous les différentes présentations (poudre libre, poudre compressée en pastilles ou en tablettes, liquide, gel, ...) actuellement proposées pour les détergents, et soit pratique d'emploi et, notamment, facile à dissoudre ou à diluer dans l'eau.

On connaît, par la Demande de Brevet Japonais n° 61-015813, un masque de beauté destiné à adoucir et hydrater la peau, qui comprend une protéine, un polysaccharide et un composant de phase aqueuse, ce dernier pouvant être soit un polyol du type glycérol, sorbitol ou polyéthylène glycol, soit un sucre comme le glucose ou le maltose.

On connaît également, par la Demande de Brevet Européen n° 0 194 097, une composition moussante pour la toilette, qui est destinée à être conditionnée dans un aérosol et qui comprend, d'une part, un concentré et, d'autre part, un gaz propulseur. Le concentré est constitué par une phase aqueuse qui contient 3 à 20% d'un agent tensio-actif anionique ou amphotère, lequel est responsable des propriétés moussantes et lavantes de la composition, 0,05 à 5% d'un agent adoucissant polymérique qui peut notamment être un polysaccharide, et 10 à 60% d'un agent hydratant pouvant, lui, être choisi, entre autres, parmi les protéines dérivées du collagène et les polyéthylène glycols.

Or, dans le cadre de leurs travaux, les Inventeurs ont constaté qu'en réunissant dans une même formulation :
- d'une part, des macromolécules hydrosolubles, de nature protéique et polyosidique, qui sont en général des composés peu ou non moussants et qui présentent des affinités spécifiques vis-à-vis des différents constituants des salissures (les protéines ayant plutôt une affinité vis-à-vis des composés protéiques et lipidiques, tandis que les polysaccharides ont une affinité préférentielle pour les sucres), tout en étant compatibles entre eux, et
- d'autre part, un ou plusieurs polyoxydes d'éthylène ou copolymères d'oxyde d'éthylène ne comportant que des motifs hydrophiles, qui ne moussent que peu ou pas et qui sont capables à la fois de rendre les salissures plus hydrophiles et, donc, plus aisément dispersables dans l'eau, et de se *"glisser"* entre lesdites salissures et la surface à laquelle elles adhèrent, facilitant ainsi leur décollement de cette surface,
il est possible d'obtenir, de manière surprenante, un détergent aux propriétés nettoyantes remarquables bien qu'exempt d'agent tensio-actif, et qui présente, par ailleurs, un pH proche de la neutralité, une très faible tendance au moussage et une aptitude au contact alimentaire. Ce détergent est, en outre, dénué d'effet néfaste pour l'environnement en raison de la biodégradabilité des éléments qui le constituent.

Les Inventeurs ont, de plus, mis en évidence qu'une formulation de ce type manifeste, en milieu aqueux, une affinité particulière pour les surfaces et une aptitude à modifier leurs comportements interfaciaux, et est donc susceptible d'être utilisée, non seulement en tant que détergent, mais de manière plus générale, en tant que surfactant.

Notamment, cette formulation s'est avérée présenter des propriétés dispersantes remarquables lors de la mise en suspension de particules minérales et organiques en milieu aqueux, se traduisant par un éclatement des amas structuraux lors de cette mise en suspension, une stabilisation des suspensions obtenues et une facilité à redisperser en milieu aqueux les agglomérats de particules obtenus après séchage desdites suspensions.

La présente Invention a donc pour objet l'utilisation d'une composition exempte d'agent tensio-actif et comprenant au moins une protéine hydrosoluble, au moins un polysaccharide hydrosoluble et au moins un composé choisi parmi les polyoxydes d'éthylène et les copolymères d'oxyde d'éthylène et d'un autre monomère hydrophile, comme surfactant

Au sens de la présente Invention, on entend par protéine ou polysaccharide *"hydrosoluble",* une protéine ou un polysaccharide apte à se dissoudre totalement dans l'eau en l'absence de tout agent ou traitement solubilisant, à une concentration pondérale au moins égale à 20 % et à une température de l'eau inférieure ou égale à 60°C.

Selon une première disposition avantageuse de l'Invention, la où les protéines présentes dans la composition sont choisies parmi des protéines ayant un point isoélectrique (pHi) compris entre 6,0 et 9,5 et, de préférence, entre 7,0 et 9.0, de manière à être amphotères à des valeurs de pH égales ou voisines à celles que présente normalement l'eau, et à avoir, ainsi, une affinité vis-à-vis de tous les composants protéiques ou osidiques des salissures, que ces composants soient cationiques ou anioniques.

Selon une autre disposition avantageuse de l'Invention, le ou les polysaccharides présents dans la composition sont choisis parmi les polysaccharides de poids moléculaire (PM) compris entre environ 10⁵ et 10⁶ et aptes à donner, une fois dissous dans l'eau, à une concentration pondérale au moins égale à 20% et à une température d'environ 25°C, une solution de viscosité d'au plus 10³ mPa.s BROOKFIELD à 100 tours/min.

Selon une disposition préférée de l'Invention, la ou les protéines et/ou le ou les polysaccharides présents dans la composition sont choisis parmi les protéines et les polysaccharides naturels.

Ainsi, la ou les protéines sont préférentiellement choisies parmi les gélatines de type A (c'est-à-dire obtenues par hydrolyse acide du collagène) et présentant une force en gelée comprise entre environ 150 et 280 Bloom BS (British Standard) telle que mesurée d'après la norme AFNOR NF V 59-001. De telles gélatines qui ont un pHi généralement compris entre 6.0 et 9.5, sont disponibles notamment auprès des Sociétés PB GELATINS, SKW BIOSYSTEMS et WEISHARDT INTERNATIONAL.

De nombreux polysaccharides naturels disponibles commercialement contiennent des protéines dont la teneur peut atteindre 10% en fonction du type de polysaccharides et de leur degré de purification. La présence de ces protéines. non seulement ne s'oppose pas à l'utilisation de ces polysaccharides pour la préparation d'une composition utilisée conformément à l'Invention, mais présente même l'avantage d'augmenter leur compatibilité avec les protéines entrant dans la constitution de cette composition.

Parmi les polysaccharides naturels, les gommes arabiques et, notamment, celles qui présentent un PM d'environ 5.10⁵ telles que commercialisées par la Société ALLAND & ROBERT sous la référence 396A. se sont révélées convenir particulièrement bien à la réalisation d'une composition conforme à l'Invention. De telles gommes présentent une teneur en protéines de l'ordre de 1 à 2%.

En variante, il est toutefois possible d'utiliser des protéines et/ou des polysaccharides de synthèse ou de biosynthèse. Notamment, des dextranes tels que les dextranes 40 et 70, qui sont commercialisés par la Société PHARMACIA & UPJOHN sous les marques Rheomacrodex® et Macrodex® , ou ceux de la Société FLUKA, peuvent avantageusement être utilisés en lieu et place des polysaccharides naturels.

Conformément à l'Invention, la composition comprend aussi au moins un composé choisi parmi les polyoxydes d'éthylène (POE) et les copolymères d'oxyde d'éthylène et d'un autre monomère hydrophile. A titre d'exemples de tels copolymères, on peut citer les copolymères blocs d'oxyde d'éthylène et de 4-vinylpyridine quatemisée tels que décrits par G. RIESS *(C.R. Académie des Sciences de Paris,* Série C, 1976, 283, pages 269 et suivantes), et les copolymères blocs d'oxyde d'éthylène et d'aminoacides obtenus à partir de polyoxydes d'éthylène aminotétéchéiiques selon le procédé décrit par H. CHERADAME *(Polymer Bulletin,* 1994, 33, pages 37 et suivantes).

Selon une autre disposition préférée de l'Invention, le ou les polyoxydes d'éthylène sont des POE de bas PM, c'est-à-dire compris entre environ 10³ et 2.10⁴, et qui sont classiquement dénommés polyéthylène glycols.

Dans le cas où l'on souhaite conférer à la composition des propriétés désincrustantes particulièrement élevées, ces propriétés peuvent être obtenues en associant ce ou ces polyoxydes d'éthylène de bas PM à un ou plusieurs polyoxydes d'éthylène de haut PM, c'est-à-dire compris entre environ 10⁵ et 5.10⁶. Toutefois, l'utilisation de POE de haut PM sera, de préférence, réservée à des applications n'impliquant aucun contact avec la peau, ni de la composition elle-même, ni des objets devant être nettoyés avec cette composition, dans la mesure où les propriétés toxicologiques des ces POE n'ont pas fait à ce jour l'objet d'études approfondies.

Conformément à l'Invention, la composition comprend, de préférence, en pourcentages en poids rapportés au poids total des composés actifs [protéine(s) + polysaccharide(s) + POE et/ou poly(oxyde d'éthylène/monomère hydrophile)] qu'elle renferme :
- 10 à 70% de protéine(s),
- 10 à 70% de polysaccharide(s), et
- 10 à 60% de POE et/ou de poly(oxyde d'éthylène/monomère hydrophile).

Selon un mode de réalisation préféré de la composition, celle-ci comprend. en pourcentages en poids rapportés au poids total des composés actifs qu'elle renferme :
- 10 à 70 % de protéine(s),
- 10 à 70% de polysaccharide(s), et
- 10 à 60% de POE de PM compris entre environ 10³ et 2.10⁴.

Dans le cas où la composition renferme également des POE de haut PM, alors celle-ci comprend, de manière préférée, en pourcentages en poids rapportés au poids total des composés actifs qu'elle renferme :
- 10 à 70 % de protéine(s),
- 10 à 70% de polysaccharide(s),
- 10 à 50% de POE de PM compris entre environ 10³ et 2.10⁴, et
- 1 à 10% de POE de PM compris entre environ 10⁵ et 5.10⁶.

Conformément à l'Invention, la composition peut comprendre, de plus, un ou plusieurs additifs choisis en fonction de l'usage auquel elle est destinée et des propriétés additionnelles que l'on souhaite lui conférer en fonction de cet usage. Ainsi, elle peut notamment comprendre :
- un ou plusieurs agents biocides, dans le cas où l'on souhaite lui donner un pouvoir assainissant, voire aseptisant ;
- une ou plusieurs charges comme des silicates, destinées à diluer les polymères actifs présents dans la composition et/ou à modifier le pH de cette dernière ;
- un ou plusieurs agents détartrants tel que l'acide citrique ;
- un ou plusieurs agents blanchissants comme les sels peroxydés ;
- un ou plusieurs agents azurants comme les pigments d'azurage ou les azureurs optiques ;
- un ou plusieurs agents vecteurs de charge ionique propres à modifier les potentiels de surface, comme les polyacrylates ou les sels d'ammonium quaternaire ;
- un ou plusieurs agents texturants comme les bentonites, les terres de diatomées ou les silices colloïdales,
- un ou plusieurs colorants et/ou parfums et/ou arômes de nature à apporter un certain agrément aux utilisateurs.

Compte tenu du risque de prolifération de micro-organismes dans des solutions et de l'aptitude que présentent ces derniers à dégrader les protéines et les polysaccharides la composition se présente, de préférence, sous forme sèche avantageusement pulvérulente.

Dans le cas où elle est préparée à partir de composés se présentant eux-mêmes sous forme sèche - ce qui est notamment le cas des gélatines, de gammes arabiques et des polyoxydes d'éthylène de poids moléculaire supérieur à 10³ , elle peut être obtenue par un simple mélange de ces composés, par exemple dans un mélangeur à poudres standards du type à pâles, à tambour ou à mouvement diagonal ou aléatoire. Dans le cas contraire, des techniques telles que celle désignée par l'anglicisme consacré par l'usage *"spray drying"* peuvent avantageusement être utilisées. Dans tous les cas. la composition sèche ainsi obtenue peut être compressée en pastilles ou en tablettes.

En variante, la composition peut se présenter sous une forme liquide ou de gel, après dissolution de ses constituants dans un volume d'eau approprié et adjonction éventuelle d'une charge stabilisante du type bentonite ou terre de diatomées et/ou d'un ou plusieurs agents bactéricides et/ou fongicides propres à empêcher la dégradation des composés qu'elle renferme.

Quelle que soit la forme sous laquelle se trouve la composition, son utilisation en tant que surfactant, présente de nombreux avantages, car :
- elle est très hydrosoluble ; aussi, sous forme sèche, elle se dissout aisément dans l'eau, tandis que, sous forme liquide, elle se dilue sans aucune difficulté ;
- elle a une tendance au moussage extrêmement réduite, voire nulle ;
- elle présente un pH neutre ou très faiblement alcalin ; ainsi, elle n'a pas ou pratiquement pas d'action corrosive ;
- elle respecte l'environnement en raison de sa biodégradabilité optimale.

De plus, lorsqu'elle est utilisée en tant que détergent :
- elle se caractérise par un fort pouvoir désincrustant et dispersant vis-à-vis des salissures bien qu'étant exempte d'agent tensio-actif ; les salissures se détachent sans difficulté de la surface des objets à nettoyer et se dispersent dans le bain de nettoyage ; toutefois, contrairement aux détergents classiques, elle est dénuée d'effet émulsifiant, ce qui évite aux salissures, une fois dispersées. de s'agglomérer les unes aux autres et de se redéposer sur les objets lors de leur retrait du bain de nettoyage, et supprime, par voie de conséquence, la nécessité de procéder à un rinçage desdits objets ;
- elle manifeste un fort pouvoir adoucissant sur les textiles et, notamment les textiles à base de laine. doublé dans ce dernier cas d'un effet anti-feutrage.

En outre, la composition présente l'avantage de pouvoir être préparée uniquement à partir de composés dont l'inocuité vis-à-vis de l'Homme et des animaux a été largement démontrée: comme les gélatines. les gommes arabiques et les polyéthylène glycols, ce qui permet. dans ce cas, d'écarter tout risque d'intoxication aiguë, même en cas d'ingestion massive. et de lui conférer une excellente tolérance cutanée.

Selon encore une autre disposition préférée de l'Invention, la composition est utilisée en tant que détergent. A titre d'exemples d'utilisation dans le domaine de la détergence, on peut citer sans que cela air un quelconque caractère limitatif:
- la détergence grand public comme le lavage manuel ou en machine de la vaisselle et du linge, y compris du linge délicat, le nettoyage des surfaces dures du type sols, murs ou plafonds, revêtements de sanitaires. vitres et glaces. surfaces de meubles telles que celles d'éléments de cuisine ou encore le nettoyage des tentures et des moquettes ;
- la détergence industrielle comme le dégraissage des métaux, la décontamination des surfaces, le nettoyage des pièces de moteurs. des cellules et carlingues d'avions, des appareillages des chaînes de fabrication ou de transport de produits alimentaires (cuves, citernes, ...), des installations et des animaux de traite ou encore le nettoyage des circuits électroniques ;
- le nettoyage des oeuvres d'art telles que peintures et sculptures, la bijouterie et la joaillerie ;
- la protection de l'environnement comme le nettoyage des surfaces polluées par des hydrocarbures (coques d'embarcations et de navires, rivages, ...).

Selon encore une autre disposition préférée de l'Invention, la composition est utilisée en tant que dispersant. A titre d'exemples d'une telle utilisation, on peut citer sans que cela ait un quelconque caractère limitatif, la mise en suspension de particules minérales ou organiques en milieu aqueux, la préparation de produits autodispersables (pour la réalisation de produits phytosanitaires par exemple) et la formulation des eaux de mouillage pour impression offset.

Selon encore une autre disposition préférée de l'Invention, la composition est utilisée pour la désémulsification du pétrole brut ou la récupération des résidus dans l'industrie minérale.

L'Invention sera mieux comprise à l'aide du complément de description qui suit et qui se réfère à des exemples de préparation de compositions aptes à être utilisées comme détergents et dispersants conformément à l'Invention et d'utilisation de ces compositions. Il va de soi, toutefois, que ces exemples sont donnés uniquement à titre d'illustrations de l'Invention et n'en constituent en aucune manière une limitation.

### EXEMPLE 1 : Préparation d'une composition apte à être utilisée comme détergent et ses utilisations

### 1.1 - Préparation

On prépare une composition se présentant sous la forme d'une poudre, en mélangeant intimement :
- 33 parties en poids de gélatine de peau de porc, de type A et de force en gelée égale à 280 Bloom BS (Société WEISHARDT INTERNATIONAL - référence F8),
- 33 parties en poids de gomme arabique atomisée, de PM moyen égal à 5.10⁵ (Société ALLAND & ROBERT - référence 396A), et
- 34 parties en poids d'un polyéthylène glycol de PM égal à 4600 (Société ALDRICH - référence 37 000-1).

La poudre ainsi obtenue peut être utilisée telle quelle en tant que détergent. En variante, elle peut être préalablement compressée sous forme de pastilles ou de tablettes, ou dissoute dans de l'eau, par exemple à une concentration comprise entre 0,5 et 5% (p/v), pour obtenir une composition détergente liquide prête à l'emploi.

### 1.2 - Utilisations

### a) Lavage manuel d'une vaisselle

On dissout la poudre préparée au point 1.1 ci-avant dans un volume d'eau chaude (40°C minimum) convenablement choisi en fonction du volume de vaisselle à laver, à raison d'une dose de 2 ml (soit environ une demi cuiller à café rase) de cette poudre par litre d'eau. On laisse reposer quelques minutes pour obtenir une dissolution totale de la poudre.

On plonge les pièces de vaisselle dans le bain ainsi obtenu. On les laisse tremper pendant quelques minutes, puis on détache les salissures desdites pièces au moyen d'une éponge ou d'une brosse. On sort les pièces de vaisselle du bain. Compte tenu d'une part, des propriétés dispersantes et non émulsifiantes de la poudre et, d'autre part, de son aptitude au contact alimentaire, les pièces de vaisselle sont mises à égoutter et à sécher sans rinçage préalable. Il est, toutefois, possible de les soumettre à un rinçage à l'eau claire, si on le souhaite.

### b) Lavage manuel de lainages

On dissout la poudre préparée au point 1.1 ci-avant dans un volume d'eau chaude (40°C minimum) convenablement choisi en fonction du volume de lainages à laver, à raison de 2 ml de cette poudre par litre d'eau. On laisse reposer quelques minutes pour obtenir une dissolution totale de la poudre.

Pour profiter des propriétés adoucissantes et anti-feutrage de la poudre, il est souhaitable d'égoutter, puis d'essorer manuellement ou en machine les lainages sans les rincer. Bien entendu, il est toutefois possible de les rincer, si on le désire.

### EXEMPLE 2 : Préparation d'une composition apte à être utilisée comme dispersant et ses utilisations

### 2.1 Préparation

On prépare une composition se présentant sous la forme d'une poudre, en mélangeant intimement :
- 20 parties en poids de gélatine de peau de porc, de type A et de force en gelée égale à 150 Bloom BS (Société SKW BIOSYSTEMS - référence PS 150),
- 50 parties en poids de gomme arabique atomisée, de PM moyen égal à 5.10⁵ (Société ALLAND & ROBERT - référence 396A), et
- 30 parties en poids d'un polyéthylène glycol de PM égal à 4600 (Société ALDRICH - référence 37 000-1).

La poudre ainsi obtenue peut être utilisée en tant que dispersant sous des formes analogues à celles mentionnées au point 1.1. ci-avant.

### 2.2 - Utilisations

### a) Empâtage de carbonates de calcium

On dissout sous agitation modérée 0.75 partie en poids de la poudre préparée au point 2.1 ci-avant dans 38,1 parties en poids d'eau chaude (40°C minimum), puis on ajoute sous vive agitation 100 parties en poids d'un mélange (50/50 : p/p) de carbonates de calcium broyés de coupures respectivement égales à 2 et 5 µm (Mikhart® 2 & 5 de la Société PROVENCALE SA).

En quelques minutes, on obtient un slurry à 72.5% ou plus d'extrait sec, de basse viscosité et d'excellente rhéologie.

### b) Empâtage de terre de diatomées

On dissout sous agitation modérée 1,5 partie en poids de la poudre préparée au point 2.1 ci-avant dans 200 parties en poids d'eau chaude (40°C minimum), puis on ajoute sous vive agitation 100 parties en poids de terre de diatomées naturelle de D50 égale à 12 µm (Primisil® 30A de la Société WORLD MINERALS EUROPE).

On obtient en quelques minutes un slurry à 35% ou plus d'extrait sec, d'excellente rhéologie et dont les particules sont parfaitement mouillées à coeur.

### c) Préparation d'un mélange autodispersable de carbonate de calcium et de talc

On dissout sous agitation modérée 1 partie en poids de la poudre préparée au point 2.1 ci-avant dans 43,5 parties en poids d'eau chaude (40°C minimum), puis on ajoute sous vive agitation 100 parties en poids d'un mélange (80/20 : p/p) de carbonate de calcium et de talc broyés de coupures respectivement égales à 2 et 8 µm (Feria® 2 de la Société BLANCS MINERAUX DE TUNISIE et Westmin® 8 de WESTMIN TALC).

En quelques minutes, on obtient un slurry à 70% ou plus d'extrait sec, de basse viscosité et d'excellente rhéologie, qui, par simple séchage à 80°C environ (par exemple, dans une étuve ou un four tunnel) permet d'obtenir un produit sec parfaitement et très facilement redispersable à plus de 70% dans de l'eau sans additif particulier.

## Revendications

1. Utilisation d'une composition exempte d'agent tensio-actif et comprenant au moins une protéine, au moins un polysaccharide et au moins un composé choisi parmi les polyoxydes d'éthylène et les copolymères d'oxyde d'éthylène et d'un autre monomère hydrophile, comme surfactant, ladite protéine et ledit polysaccharide étant aptes à se dissoudre totalement dans l'eau en l'absence de tout agent ou traitement solubilisant, à une concentration pondérale au moins égale à 20 % et à une température de l'eau inférieure ou égale à 60°C.

2. Utilisation selon la revendication 1, caractérisée en ce la ou les protéines sont choisies parmi des protéines ayant un point isoélectrique (pHi) compris entre 6,0 et 9,5 et, de préférence, entre 7,0 et 9,0.

3. Utilisation selon la revendication 1 ou la revendication 2, **caractérisée en ce que** le ou les polysaccharides sont choisis parmi les polysaccharides de poids moléculaire (PM) compris entre environ 10⁵ et 10⁶ et aptes à donner, une fois dissous dans l'eau, à une concentration au moins égale à 20% en poids et à une température d'environ 25°C, une solution de viscosité, d'au plus 10³ mPa.s BROOKFIELD à 100 tours/min.

4. Utilisation selon l'une quelconque des revendications précédentes, **caractérisée en ce que** la ou les protéines et/ou le ou les polysaccharides sont choisis parmi les protéines et les polysaccharides naturels.

5. Utilisation selon l'une quelconque des revendications précédentes, **caractérisée en ce que** la ou les protéines sont choisies parmi les gélatines de type A et présentant une force en gelée comprise entre environ 150 et 280 Bloom BS (British Standard) telle que mesurée d'après la norme AFNOR NF V 59-001.

6. Utilisation selon l'une quelconque des revendications précédentes, **caractérisée en ce que** le ou les polysaccharides sont choisis parmi les gommes arabiques.

7. Utilisation selon la revendication 6, **caractérisée en ce que** le ou les polysaccharides sont choisis parmi les gommes arabiques qui présentent un PM moyen d'environ 5.10⁵.

8. Utilisation selon l'une quelconque des revendications précédentes, **caractérisée en ce que** la composition comprend un ou plusieurs POE de PM compris entre environ 10³ et 2.10⁴.

9. Utilisation selon la revendication 8, **caractérisée en ce que** la composition comprend de plus un ou plusieurs POE de PM compris entre environ 10⁵ et 5.10⁶.

10. Utilisation selon l'une quelconque des revendications précédentes, **caractérisée en ce que** la composition comprend, en pourcentages en poids rapportés au poids total des composés actifs [protéine(s) + polysaccharide(s) + POE et/ou poly(oxyde d'éthylène / monomère hydrophile)] qu'elle renferme :
- 10 à 70% de protéine,
- 10 à 70% de polysaccharide, et
- 10 à 60% de POE et/ou de poly(oxyde d'éthylène/monomère hydrophile).

11. Utilisation selon l'une quelconque des revendications précédentes, **caractérisée en ce que** la composition comprend, en pourcentages en poids rapportés au poids total des composés actifs [protéine(s) + polysaccharide(s) + POE et/ou poly(oxyde d'éthylène / monomère hydrophile)] qu'elle renferme :
- 10 à 70 % de protéine,
- 10 à 70% de polysaccharide, et
- 10 à 60% de POE de PM compris entre environ 10³ et 2.10⁴.

12. Utilisation selon l'une quelconque des revendications 1 à 10, **caractérisée en ce que** la composition comprend, en pourcentages en poids rapportés au poids total des composés actifs [protéine(s) + polysaccharide(s) + POE et/ou poly(oxyde d'éthylène / monomère hydrophile)] qu'elle renferme :
- 10 à 70 % de protéine,
- 10 à 70% de polysaccharide, et
- 10 à 50% de POE de PM compris entre environ 10³ et 2.10⁴, et
- 1 à 10% de POE de PM compris entre environ 10⁵ et 5.10⁶.

13. Utilisation selon l'une quelconque des revendications précédentes, **caractérisée en ce que** la composition comprend, de plus, un ou plusieurs additifs parmi les agents biocides, les charges, les agents détartrants, les agents blanchissants, les agents azurants, les agents vecteurs de charge ionique, les agents texturants, les colorants et les parfums.

14. Utilisation selon l'une quelconque des revendications précédentes, **caractérisée en ce que** la composition est utilisée comme détergent.

15. Utilisation selon la revendication 14, **caractérisée en ce que** la composition est utilisée pour le lavage manuel ou en machine de la vaisselle et du linge, le nettoyage des surfaces dures du type sols, murs ou plafonds, revêtements de sanitaires, surfaces de meubles, vitres et glaces, le nettoyage des tentures et des moquettes, le dégraissage des métaux, le nettoyage des pièces de moteurs, des cellules et carlingues d'avion, des appareillages des chaînes de fabrication ou de transport de produits alimentaires, des installations et des animaux de traite, des surfaces polluées par des hydrocarbures, des circuits électronique, le nettoyage des oeuvres d'art telles que peintures et sculptures ou pour la bijouterie et la joaillerie.

16. Utilisation selon l'une quelconque des revendications 1 à 13, **caractérisée en ce que** la composition est utilisée comme dispersant.

17. Utilisation selon la revendication 16, **caractérisée en ce que** la composition est utilisée pour la mise en suspension de particules minérales ou organiques en milieux aqueux, la préparation de produits autodispersables ou pour la formulation des eaux de mouillage pour impression offset.

18. Utilisation selon l'une quelconque des revendications 1 à 13, **caractérisée en ce que** la composition est utilisée pour la désémulsification du pétrole brut ou pour la récupération des résidus dans l'industrie minérale.

## Claims

1. Use of a composition free from surface-active agent and comprising at least one protein, at least one polysaccharide and at least one compound chosen from amongst ethylene polyoxides and copolymers of ethylene oxide and another hydrophilic monomer, as a surfactant, the said protein and the said polysaccharide being able to dissolve completely in water in the absence of any solubilising agent or treatment, at a concentration by weight of at least 20% and at a water temperature of less than or equal to 60°C.

2. Use according to Claim 1, **characterised in that** the protein or proteins are chosen from amongst proteins having an isoelectric point (pHi) between 6.0 and 9.5 and preferably between 7.0 and 9.0.

3. Use according to Claim 1 or Claim 2, **characterised in that** the polysaccharide or polysaccharides are chosen from amongst the polysaccharides with a molecular weight (MW) of between approximately 10⁵ and 10⁶ and able to give, once dissolved in water, at a concentration of at least 20% by weight and at a temperature of approximately 25°C, a solution with a viscosity of no more than 10³ mPa.s Brookfield at 100 revolutions/min.

4. Use according to any one of the preceding claims, **characterised in that** the protein or proteins and the polysaccharide or polysaccharides are chosen from amongst natural proteins and polysaccharides.

5. Use according to any one of the preceding claims, **characterised in that** the protein or proteins are chosen from amongst gelatins of type A and having a gel strength of between approximately 150 and 280 Bloom BS (British Standard) as measured in accordance with AFNOR NF V 59-001.

6. Use according to any one of the preceding claims, **characterised in that** the polysaccharide or polysaccharides are chosen from amongst the gums arabic.

7. Use according to Claim 6, **characterised in that** the polysaccharide or polysaccharides are chosen from amongst the gums arabic which have a mean MW of approximately 5.10⁵.

8. Use according to any one of the preceding claims, **characterised in that** the composition comprises one or more EPOs with an MW of between approximately 10³ and 2.10⁴.

9. Use according to Claim 8, **characterised in that** the composition also comprises one or more EPOs with an MW of between approximately 10⁵ and 5.10⁶.

10. Use according to any one of the preceding claims, **characterised in that** the composition comprises, in percentages by weight referred to the total weight of active compounds (protein(s) + polysaccharide(s) + EPO and/or ethylene polyoxide/hydrophilic monomer) which it contains:
- 10% to 70% protein,
- 10% to 70% polysaccharide, and
- 10% to 60% EPO and/or ethylene polyoxide/hydrophilic monomer.

11. Use according to any one of the preceding claims,
**characterised in that** the composition comprises, in percentages by weight referred to the total weight of active compounds (protein(s) + polysaccharide(s) + EPO and/or ethylene polyoxide/hydrophilic monomer) which it contains:
- 10% to 70% protein,
- 10% to 70% polysaccharide, and
- 10% to 60% EPO with an MW of between approximately 10³ and 2.10⁴.

12. Use according to any one of Claims 1 to 10, **characterised in that** the composition comprises, in percentages by weight referred to the total weight of active compounds (protein(s) + polysaccharide(s) + EPO and/or ethylene polyoxide/hydrophilic monomer) which it contains:
- 10% to 70% protein,
- 10% to 70% polysaccharide, and
- 10% to 50% EPO with an MW of between approximately 10³ and 2.10⁴, and
- 1% to 10% EPO with an MW of between approximately 10⁵ and 5.10⁶.

13. Use according to any one of the preceding claims, **characterised in that** the composition also comprises one or more additives from amongst biocides, fillers, descaling agents, bleaching agents, brighteners, ion charge carriers, texturing agents, colouring agents and perfumes.

14. Use according to any one of the preceding claims, **characterised in that** the composition is used as a detergent.

15. Use according to Claim 14, **characterised in that** the composition is used for the manual or machine washing of dishes or laundry, the cleaning of hard surfaces of the floor, wall or ceiling type, bathroom and toilet surfaces, furniture surfaces, windows and mirrors, the cleaning of curtains and carpets, the degreasing of metals, the cleaning of aircraft engine parts, airframes and cabins, equipment on production lines or for the conveyance of food products, milking installations and animals, surfaces contaminated by hydrocarbons, electronic circuits, the cleaning of works of art such as paintings and sculptures or for jewellery.

16. Use according to any one of Claims 1 to 13, **characterised in that** the composition is used as a dispersant.

17. Use according to Claim 16, **characterised in that** the composition is used for putting mineral or organic particles in suspension in an aqueous medium, for the preparation of auto-dispersing products or for the formulation of wetting water for offset printing.

18. Use according to any one of Claims 1 to 13, **characterised in that** the composition is used for the demulsification of crude oil or for the recovery of residues in the mineral industry.

## Patentansprüche

1. Verwendung einer Zusammensetzung., die kein oberflächenaktives Mittel enthält und wenigstens ein Protein, wenigstens ein Polysaccharid und wenigstens eine aus Ethylenpolyoxiden und Copolymeren von Ethylenoxid und einem weiteren hydrophilen Monomer ausgewählte Mischung, wobei das genannte Protein und das genannte Polysaccharid als Tensid zur vollständigen Auflösung in Wasser in Abwesenheit von irgendeinem Lösungsvermittler oder irgendeiner Behandlung zur Lösungsvermittlung bei einem Gewichtsanteil von wenigstens gleich 20 % und einer Wassertemperatur von weniger als oder gleich 60° C.

2. Verwendung gemäß Anspruch 1, **dadurch gekennzeichnet, daß** das oder die Proteine aus Proteinen ausgewählt sind, die einen isoelektrischen Punkt (pHi) im Bereich von 6,0 bis 9,5 besitzen und, bevorzugt, in einem Bereich von 7,0 bis 9,0.

3. Verwendung gemäß Anspruch 1 oder Anspruch 2, **dadurch gekennzeichnet, daß** das oder die Polysaccharide aus Polysacchariden mit einem Molekulargewicht (MG) im Bereich von ungefähr 10⁵ bis 10⁶ ausgewählt sind und fähig sind, einmal in Wasser gelöst, bei einer Konzentration von wenigstens gleich 20 Gew.-% und bei einer Temperatur im Bereich von 25° C eine Lösung mit einer Viskosität von größer 10³ mPa.s BROOKFIELD bei 100 Umdrehungen/min. zu ergeben.

4. Verwendung gemäß einem der vorhergehenden Ansprüche, **dadurch gekennzeichnet, daß** das oder die Proteine und/oder das oder die Polysaccharide aus natürlichen Proteinen und Polysacchariden ausgewählt sind.

5. Verwendung gemäß einem der vorhergehenden Ansprüche, **dadurch gekennzeichnet, daß** das oder die Proteine ausgewählt sind aus Gelantinen vom Typ A und eine Gelierkraft im Bereich von ungefähr 150 bis 280 Bloom BS (British Standard) aufweisen, gemessen nach der Norm AFNOR NF V 59-001.

6. Verwendung gemäß einem der vorhergehenden Ansprüche, **dadurch gekennzeichnet, daß** das oder die Polysaccharide aus verschiedenen Sorten Gummi Arabicum ausgewählt sind.

7. Verwendung gemäß Anspruch 6, **dadurch gekennzeichnet, daß** das oder die Polysaccharide aus Gummi Arabicum Sorten ausgewählt sind, die ein mitteleres MG von ungefähr 5•10⁵ aufweisen.

8. Verwendung gemäß einem der vorhergehenden Ansprüche, **dadurch gekennzeichnet, daß** die Zusammensetzung ein oder mehrere Ethylenpolyoxide (POE) mit einem MG in einem Bereich von ungefähr 10³ bis 2•10⁴ umfaßt.

9. Verwendung gemäß Anspruch 8, **dadurch gekennzeichnet, daß** die Zusammensetzung ferner einen oder mehrere Ethylenpolyoxide (POE) mit einem MG in einem Bereich von ungefähr 10⁵ bis 5•10⁶ umfaßt.

10. Verwendung gemäß einem der vorhergehenden Ansprüche, **dadurch gekennzeichnet, daß** die Zusammensetzung, in Gew.-% bezogen auf das Gesamtgewicht der aktiven Mischungen [Protein(e) + Polysaccharid(e) + Ethylenpolyoxid (POE) und/oder Ethylenpoly(oxid/hydrophiles Monomer], enthält:
- 10 bis 70 % Protein,
- 10 bis 70 % Polysaccharid und
- 10 bis 60 % Ethylenpolyoxid (POE) und/oder Ethylenpoly(oxid/hydrophiles Monomer).

11. Verwendung gemäß einem der vorhergehenden Ansprüche, **dadurch gekennzeichnet, daß** die Zusammensetzung, in Gew.-% bezogen auf das Gesamtgewicht der aktiven Mischungen [Protein(e) + Polysaccharid(e) + Ethylenpolyoxid (POE) und/oder Ethylenpoly(oxid/hydrophiles Monomer], enthält:
- 10 bis 70 % Protein,
- 10 bis 70 % Polysaccharid und
- 10 bis 60 % Ethylenpolyoxid (POE) mit einem MG in einem Bereich von ungefähr 10³ bis 2•10⁴.

12. Verwendung gemäß einem der Ansprüche 1 bis 10, **dadurch gekennzeichnet, daß** die Zusammensetzung, in Gew.-% bezogen auf das Gesamtgewicht der aktiven Mischungen [Protein(e) + Polysaccharid(e) + Ethylenpolyoxid (POE) und/oder Ethylenpoly(oxid/hydrophiles Monomer], enthält:
- 10 bis 70 % Protein,
- 10 bis 70 % Polysaccharid und
- 10 bis 50 % Ethylenpolyoxid (POE) mit einem MG in einem Bereich von ungefähr 10³ bis 2•10⁴ und
- 1 bis 10 % Ethylenpolyoxid (POE) mit einem MG in einem Bereich von ungefähr 10⁵ bis 5•10⁶.

13. Verwendung gemäß einem der vorhergehenden Ansprüche, **dadurch gekennzeichnet, daß** die Zusammensetzung ferner einen oder mehrere Zusätze ausgewählt aus bioziden Agenzien, Beladungsmitteln, Entkalkungsmitteln, Bleichmitteln, Bläuungsmitteln, Agenzien zur Übertragung von ionischen Ladungen, Texturagenzien, Farbmitteln und Parfums enthält.

14. Verwendung gemäß einem der vorhergehenden Ansprüche, **dadurch gekennzeichnet, daß** die Zusammensetzung als Detergens verwendet wird.

15. Verwendung gemäß Anspruch 14, **dadurch gekennzeichnet, daß** die Zusammensetzung zur manuellen oder zur Maschinenwäsche von Geschirr und Wäsche verwendet wird, dem Reinigen von harten Oberflächen von der Art von Böden, Mauern oder Decken, Sanitärverkleidungen, Möbeloberflächen, Fenstern und Spiegeln, dem Reinigen von Wandbespannungen und Teppichböden, dem Entfetten von Metallteilen, dem Reinigen von Motorteilen, von Flugzeugkabinen und -cockpits, von Fließbandvorrichtung zur Herstellung oder zum Transport von Lebensmitteln, von Molkereieinrichtungen oder Milchvieh, von mit Kohlenwasserstoffen verschmutzten Oberflächen, von elektronischen Schaltkreisen, der Reinigung von Kunstwerken, wie Bildern und Skulpturen oder für Schmuck und Juwelen.

16. Verwendung gemäß einem der Ansprüche 1 bis 13, **dadurch gekennzeichnet, daß** die Zusammensetzung als Dispersionsmittel verwendet wird.

17. Verwendung gemäß Anspruch 16, **dadurch gekennzeichnet, daß** die Zusammensetzung zur Suspendierung von mineralischen oder organischen Partikeln in wässriger Umgebung verwendet wird, zur Herstellung von selbstdispergierenden Produkten oder zur Formulierung von Benetzungsmitteln für den Offsetdruck.

18. Verwendung gemäß einem der Ansprüche 1 bis 13, **dadurch gekennzeichnet, daß** die Zusammensetzung zum Deemulgieren von Rohöl verwendet wird oder zur Rückgewinnung von Rückständen der Mineralstoffindustrie.
